(19) Europäisches Patentamt
European Patent Office
Office européen des brevets

(11) **EP 3 499 510 A1**

(12) **EUROPEAN PATENT APPLICATION**

(43) Date of publication:
**19.06.2019 Bulletin 2019/25**

(51) Int Cl.:
***G16H 40/63*** (2018.01)     ***A61B 5/00*** (2006.01)
***G16H 50/30*** (2018.01)

(21) Application number: **17207468.4**

(22) Date of filing: **14.12.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **Koninklijke Philips N.V.
5656 AE Eindhoven (NL)**

(72) Inventors:
• **WEFFERS-ALBU, Mirela Alina
5656 AE Eindhoven (NL)**
• **DUNIAS, Paraskevas
5656 AE Eindhoven (NL)**

(74) Representative: **de Haan, Poul Erik
Philips International B.V.
Philips Intellectual Property & Standards
High Tech Campus 5
5656 AE Eindhoven (NL)**

(54) **SYSTEM AND METHOD FOR MONITORING WOUND HEALING**

(57)     There is provided a system (100) for monitoring wound healing of a subject. The system includes a strip (102) for covering a region of interest of the subject comprising one or more sensing means (104, 106) being in contact with the region of interest; wherein the region of interest comprises a wound (108), and wherein the one or more sensing means (104, 106) is configured for detecting one or more parameters of the wound (108). The system (100) also includes an image sensing module (110) for capturing an image of the region of interest. The system (100) also includes a calculation module (112) for calculation of a wound healing score based on the parameters detected by the one or more sensing means (104, 106) and the image acquired by the image sensing module (110), said wound healing score indicating a status of the wound healing. The system (100) further includes an output module (118) for outputting the calculated wound healing score. A computer-implemented method and a computer program product are also disclosed.

Fig. 1

**Description**

FIELD OF THE INVENTION

The invention relates to a system and method for monitoring wound healing of a subject.

BACKGROUND TO THE INVENTION

Wound healing is a complex and dynamic process of replacing devitalized and missing cellular structures and tissue layers. In undamaged skin, the surface layer and deeper layer form a protective barrier against the external environment. When the barrier is broken, a regulated sequence of biochemical events is set into motion to repair the damage. This process is divided into predictable phases: blood clotting, i.e.hemostasis, inflammation, tissue growth, and tissue remodeling.

The wound healing process is not only complex but also fragile, and it is susceptible to interruption or failure leading to the formation of non-healing chronic wounds. Factors that contribute to non-healing chronic wounds are diabetes, venous or arterial disease, infection, and metabolic deficiencies of old age. The population is confronted with societal and medical trends that impact the healthcare system regarding wound care - the population is progressively aging which leads to an increase in prevalence of diabetes and cardiovascular diseases. This translates into the high wound prevalence and corresponding high wound care costs.

Current medical practice is confronted with a number of challenges coming from the fact that wound care is not a discrete medical specialty and often relies on personal experience with wound treatment and opinion of colleagues. In that sense quality of wound care is hampered by lack of knowledge education about basic wound evaluation/assessment, wound treatments and management practices, wound prevention protocols, use of advanced supplies and treatment modalities, and lack of standardization of wound measurements, wound outcomes/metrics, wound quality reporting, etc.

In addition to the high cost associated with wound care, quality of wound care is paramount for ensuring proper healing. Inadequate care (e.g. late recognition and intervention of infections) have severe impact on the patient leading to limb amputations and death.

The current practice has the disadvantage of high costs and insufficient wound care quality. Therefore, it would be desirable to have solution that provides improved wound healing monitoring system that result in high quality of wound care.

SUMMARY OF THE INVENTION

According to a first aspect, the present invention provides a system for monitoring wound healing of a subject, the system comprising a strip for covering a region of interest of the subject comprising one or more sensing means being in contact with the region of interest; wherein the region of interest comprises a wound, and wherein the one or more sensing means is configured for detecting one or more parameters of the wound; an image sensing module for capturing an image of the region of interest; a calculation module for calculation of a wound healing score based on the parameters detected by the one or more sensing means and the image acquired by the image sensing module, said wound healing score indicating a status of the wound healing and an output module for outputting the calculated wound healing score.

By combining data derived from both sensing means having direct contact with the wound and the image sensing module without direct contact with the wound, an accurate wound healing status and progress can be assessed. Thus, the assessment is more precise and more objective. Furthermore, the assessment can be made relatively quickly, and in real time.

In some embodiments, the one or more sensing means comprises a first sensing means for detecting PH level of the wound and a second sensing means for detecting temperature level of the wound.

In some embodiments, the colour of the first sensing means is altered based on the detected PH level of the wound, and the colour of the second sensing means is altered based on detected temperature level of the wound.

In some embodiments, the system further comprises an image processing module for processing the image acquired by the image sensing module to derive a plurality of factors contributing to the determination of the wound healing score.

In some embodiments, the plurality of factors comprises a Pulse Oximeter Oxygen Saturation (SpO2) level of the wound; a hydration level of the wound; a haematocrit level of the wound; the colour of the first and the second sensing means; the location of the wound within the region of interest of the subject; the size of the wound; the maximum width of the wound; the maximum height of the wound; the coloration of the wound.

In some embodiments, the system further comprises a wound infection determination module configure for calculating a wound infection risk score indicating the wound infection risk. In some embodiments, the wound infection risk score is calculated by the wound infection determination module based on at least one of the following factors, a haematocrit level of the wound determined by the image processing module; a PH level of the wound detected by the first sensing means; a temperature level of the wound detected by the second sensing means. In some embodiments, a weight is assigned to the at least one of the haematocrit level of the wound, the PH level of the wound, and the temperature level of the wound, wherein the weight of a given parameter is determined based on the contribution of the given parameter to the cause of the wound infection.

In some embodiments, the wound healing score is determined based on at least one of following factors: the Pulse Oximeter Oxygen Saturation (SpO2) level of the wound; the hydration level of the wound; the wound infection risk; the size of the wound; the maximum width of the wound; the maximum height of the wound; the coloration of the wound. By taking the measured data all of the factors of the plurality of factors, the determination of the wound healing status can be more accurate and more objective. Thus, a better assessment of wound healing process can be achieved.

In some embodiments, a weight is assigned to the at least one of the following parameters: the Pulse Oximeter Oxygen Saturation (SpO2) level of the wound; the hydration level of the wound; the wound infection risk; the size of the wound; the maximum width of the wound; the maximum height of the wound; and the coloration of the wound, wherein the weight of a given parameter is determined based on the contribution of the given parameter to the impact of the wound healing. By weighting the factors according to importance, or according to their relative contribution to the wound healing process, the overall risk of wound healing can be more relevant.

The strip may, in some embodiments, be a transparent strip or semi-transparent strip configured for allowing the wound being visible and detectable by the image sensing module. This enables the wound being monitored by the image sensing module in real time and in unobtrusive manner.

In some embodiments, the image sensing module comprises a light source configured for emitting a light signal with various wavelengths to the region of interest; an infrared camera configured for detecting one or more parameters of the wound.

In some embodiments, the system further comprises a communication module configured for transmitting information to a second system including at least one of the wound healing score of the subject; the wound healing score of the subject over a period of time; suggestion or alert message corresponding to the wound healing score of the subject. This allows a real time data transmission from the patient to the medical professional and enables the quick reaction to the risk situation.

According to a second aspect, the present invention provides a computer-implemented method for monitoring wound healing of a subject. The method comprises detecting one or more parameters of a wound within a region of interest of the subject by one or more sensing means being in contact with the region of interest; capturing an image of the region of interest by an image sensing module; and calculating a wound healing score based on the parameters detected by the one or more sensing means and the image acquired by the image sensing module, wherein the wound healing score indicating a status of the wound healing; outputting the calculated wound healing score.

According to a third aspect, the present invention provides an computer program product comprising instructions for causing a processor system to perform the steps of detecting one or more parameters of a wound within a region of interest of the subject by one or more sensing means being in contact with the region of interest; capturing an image of the region of interest by an image sensing module; and calculating a wound healing score based on the parameters detected by the one or more sensing means and the image acquired by the image sensing module, wherein the wound healing score indicating a status of the wound healing; outputting the calculated wound healing score.

It will be appreciated by those skilled in the art that two or more of the above-mentioned embodiments, implementations, and/or optional aspects of the invention may be combined in any way deemed useful. Modifications and variations of the system, the computer-implemented method, and/or the computer program product, which correspond to the described modifications and variations of the system, can be carried out by a person skilled in the art on the basis of the present description.

BRIEF DESCRIPTION OF THE DRAWINGS

For a better understanding of the invention, and to show more clearly how it may be carried into effect, reference will now be made, by way of example only, to the accompanying drawings, in which:

Figure 1 is a schematic illustration of an example of a system for monitoring the wound healing of a subject, according to embodiments of the invention;

Figure 2 is a flowchart of an example of a computer-implemented method for monitoring the wound healing of a subject, according to embodiments of the invention;

Figure 3 shows a computer readable medium comprising instructions for causing a processor system to perform the computer-implemented method.

It should be noted that the figures are purely diagrammatic and not drawn to scale. In the Figures, elements which correspond to elements already described may have the same reference numerals.

DETAILED DESCRIPTION OF EMBODIMENTS

The present disclosure provides an improved system for monitoring wound healing of a subject. The term "subject", in the context of this disclosure may be considered to be any person. The subject does not need to be located in a medical environment, such as in a hospital; however, the system may be implemented within a medical institution, and subjects located in such a medical institution may be referred to as patients.

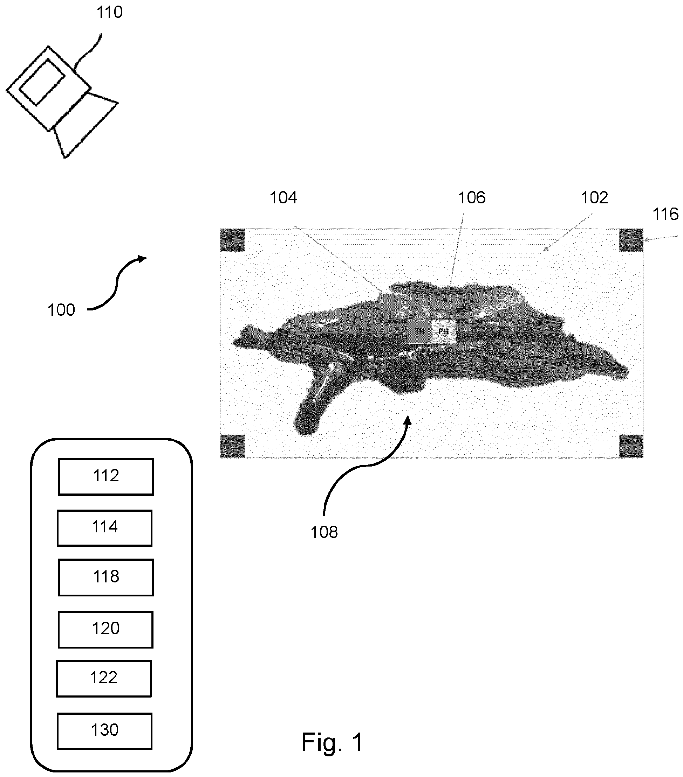

Fig. 1 shows, schematically, an example of a system 100 which is configured for monitoring wound healing.

The system 100 may comprise a strip 102 for covering a region of interest of the subject comprising one or more sensing means (104, 106) being in contact with the region of interest. The strip 102 may be any types of patch, bandage or strip made by any types of materials and with various shapes that is suitable for attaching to the skin of the wounded subject. The region of interest comprises a wound 108. The strip 102 may comprises four markers 116 located at each corner of the strip 102. In an embodiment, the strip 102 may be a transparent strip or semi-transparent strip configured for allowing the wound 108 being visible and detectable by the system 100. The one or more sensing means (104, 106) is configured for detecting one or more parameters of the wound 108. The sensing means may be any types of sensors that are suitable for being in contact with the wound 108 for measuring one or more parameters of the wound 108. In an embodiment, the sensing means 104 is a senor for detecting PH level of the wound 108; and the sensing means 106 is a sensor for detecting temperature level of the wound 108. In an embodiment, the sensing means (104, 106) may be a sensor based textile patch configured for detecting PH level and temperature level respectively. In an embodiment, the colour of the first sensing means 104 may be altered based on the detected PH level of the wound, and the colour of the second sensing means 106 may be altered based on detected temperature level of the wound.

In an embodiment, the sensor based textile patch (104, 106) may alter colours in the following way: the textile is infused with dye that reacts to different pH values. Healthy skin and healed wounds usually show a pH value of or below 5. If the PH value increases, namely shifting from the acid into the alkaline range, it indicates tissue progress deterioration in the wound healing process. PH values between 6.5 and 8.5 indicate an onset and development of infection. The textile changes colour from green to red in case of increasing PH. Increasing amounts of red of the sensor based textile patch colour indicate wound deterioration. Red tints correspond to PH values between 6.5-8.5 indicating infection onset and further infection development. The thermochromic textile patch that changes colour based on temperature levels, namely the colour ranges from white to red within the range 35 to 38 degrees Celsius. Decreased wound temperature, for instance below 36 degrees Celcius, will impede healing.

In an embodiment, the four markers 116 placed at the corners of the strip 102 are used for geometrical alignment, orientation and scaling, as well as illumination and colour calibration/alignment. Based on the placement of markers, it is possible to scale and calibrate the colour of the wound itself as well as the colours of the PH and TH patches.

The system 100 may further comprise an image sensing module 110 for capturing an image of the region of interest of the subject. The image sensing module 110 may comprise an infrared camera or any types of vital sign camera that is capable of detecting various parameters of the wound, among others, the tissue oxygenation of the wound, the tissue hydration of the wound, the Hematocrit values of the wound, etc. The image sensing module 10 may further comprise a light source configured for emitting a light signal with various wavelengths to the region of interest.

The system 100 may further comprise an image processing module 114 for processing the image acquired by the image sensing module 110 to derive a plurality of factors contributing to the determination of the wound healing score. The plurality of factors comprises a Pulse Oximeter Oxygen Saturation (SpO2) level of the wound 108; a hydration level of the wound (108); a haematocrit level of the wound (108); the colour of the first and the second sensing means (104, 106); the location of the wound (108) within the region of interest of the subject; the size of the wound (108); the maximum width of the wound (108); the maximum height of the wound (108); the coloration of the wound (108).

In an embodiment, the image processing module 114 may be configured for performing remote image spectrometry analysis to determine the level of SpO2 in the wound which contributes to determine the wound healing score. SpO2 indicates the percentage of hemoglobin (which carries O2) present in blood. Generally speaking, the higher the SpO2, the better the healing process would be. Accordingly, low tissue oxygen levels may compromise wound healing and resistance to infection.

The determination of the level of SpO2 may follow with a similar method as indicated in US 5277181 A "Noninvasive measurement of hematocrit and hemoglobin content by differential optical analysis" and "Masimo signal extraction pulse oximetry ", J. M. Goldman et. al., Journal of Clinical Monitoring and Computing, vol 16, No 7 2000, where the tissue is illuminated at two light wavelengths, e.g. 630nm and 940nm, and the amount of scattered light in the tissue in both cases is detected after filtering out the specular and diffuse surface reflections. The SpO2 level is indicated by the ratio of the calculated light extinction corresponding to tissue illumination at each light wavelengths. This ratio is related to the SpO2 level of arterial blood as well as the extinction coefficients of arterial blood which are calibrated based on population samples measurements.

In an embodiment, the image processing module 114 may be configured for detecting hydration level of the wound 108 which contributes to determine the wound healing score. This is based on the insight that water is the primary way that oxygen and nutrients are delivered directly to the wound bed to support the healing process which makes proper tissue hydration highly important for recovery. In that sense, dehydration is one of the most common reasons why cell function becomes disrupted. In other words, if an injury does not maintain proper moisture, epithelial cells that work to migrate over repaired tissue will not be able to cover the wound at a normal pace, leaving it more susceptible to open air and infection. The image processing module 114 may detect tissue hydration by using a similar remote image spectrometry analysis as described above for the detection of SpO2 levels with the difference that the two light source wavelengths used this time are 630nm and 1225nm. Tissue hydration levels are given by the ratio between the light extinction (corresponding to tissue illumination at each light wavelengths). In this calculation it is expected that SpO2 level is stable and constant.

In the situations where both SpO2 and hydration are simultaneously varying, the image processing module 114 may be configured for combining both measurements as mentioned above to simultaneously detect SpO2 as well as hydration of the wound. Therefore, the image processing unit 114 may sequentially illuminate the area of interest by three light sources at different wavelengths, e.g. 630nm, 940nm, 1225nm. In one embodiment, the image processing unit 114 may calculate two ratios as described above based on the measurements on 630nm and 1225nm as well as based on the measurement on 630nm and 940nm. In the next step, a two-dimensional, namely SpO2 variations and hydration variations, calibration may be performed based on population samples.

In an embodiment, the image processing module 114 may be configured for detecting hematocrit levels which contributes to determine the wound healing score. The detection may be done using a similar remote image spectrometry analysis method described above for the detection of SPO2 and hydration levels as indicated above. The HematocritLevel may be calculated as equal to 1 / HydrationLevel.

In an embodiment, the image processing module 114 may be configured for determining wound size, area, and coloration of the wound and their trends. In an embodiment, the following parameters may be used for such determination: Delta_SizeX, Delta_SizeY, Delta_WoundArea, Delta_WoundRed, where SizeX indicates the maximum width of wound area, SizeY indicates the maximum hight of the wound area, WoundArea indicates the area of the wound, WoundRed indicates the amount of red colour detected in the wound bed after calibration based on the corner markers.

The system may further comprise a wound infection determination module 120 for calculating a wound infection risk score indicating the wound infection risk. The wound infection risk score may be calculated by the wound infection determination module 120 based on at least one of the following factors a haematocrit level of the wound 108 determined by the image processing module 114; a PH level of the wound 108 detected by the first sensing means 104; a temperature level of the wound 108 detected by the second sensing means 106.

In an embodiment, the Hematocrit levels and evolution trends may be detected by using a similar remote image spectrometry analysis method described above for the detection of SpO2 and hydration levels as indicated above. The Hematocrit Level may be calculated as equal to 1 / HydrationLevel. Hematocrit levels may contribute to the determination of the infection risk. This is based on the insight that low or decreasing levels of hematocrit would indicate anemia and/or infection.

In an embodiment, the temperature values and evolution trends may be detected by performing video processing techniques via the image processing module 114 to detect the colour changes of thermochromic textile patch based on temperature levels, i.e. the colour ranges from white to red within the range 35 to 38 degrees Celsius. This is based on the insight that decreased wound temperature, e.g. below 36 degrees Celcius, would impede healing. The detection of the patch (104, 106) is accomplished based on detection of at least three markers 116 indicating dressing corners and the standard position of the patch at the diagonals intersection, of the overall dressing with a pre-determined shift to right.

In an embodiment, after image/colour calibration, the amount of red colour in the patch (104, 106) may be recorded in order to determine trends over time.

Optionally, the system 100 may further comprises a pyrometric thermal sensor to determine the wound bed temperature.

In an embodiment, the PH levels and evolution trends may be detected by performing video processing techniques via the image processing module 114 to detect the colour changes of the sensor based textile patch based on PH levels. The textile changes colour from green to red in case of increasing pH. Increasing amounts of red of the sensor based textile patch colour may indicate wound deterioration. The detection of patch (104, 106) is accomplished based on detection of the at least three markers indicating dressing corners and the standard position of the patch, e.g. at the diagonals intersection, of the overall dressing with a shift to right. After image/colour calibration, the amount of red colour in the patch (104, 106) may be recorded by the wound infection determination module 120 in order to determine trends over time.

In some embodiments, the combination of determined haematocrit level of the wound (referred to as "HematocritLevel"), the temperature level of the wound (referred to as "TemperatureLevel") and the PH level of the wound (referred to as "PHLevel') may be used to calculate an infection risk score (referred to as "InfectionRiskScore"). For example, the infection risk score may be calculated according to the following formula:

$$InfectionRiskScore = HematocritLevel \otimes TemperatureLevel \otimes PHLevel \qquad [1]$$

where $\otimes$ represents a generic symbol, meaning that the terms may be combined in some way.

In an embodiment, a weight may be assigned to the at least one of the haematocrit level of the wound, the PH level of the wound, and the temperature level of the wound. The weight of a given parameter is determined based on the contribution of the given parameter to the cause of the wound infection. In some embodiments, if, over a period of time, the temperature level of the wound follows a trend to increase rapidly, then this might be a significant contributory factor to the wound healing. Thus, the factor TemperatureLevel may be weighted heavily compared to the factor HaematocritLevel.

Thus, in some particular embodiments, the infection risk score may be calculated according to the following formula:

$$InfectionRiskScore = HematocritLevel*coeff1*weight1 + TemperatureLevel*$$
$$coeff1*weight2 + PHLevel* coeff1*weight3 \qquad [2]$$

where HematocritLevel, TemperatureLevel, PHLevel represent current values of these parameters, where coeff1*weight 1 is the weight applied to the Hematocrit Level, coeff1*weight2 is the weight applied to the Temperature Level, and coeff1*weight 3 is the weight applied to the PH Level.

The weights are equal to the absolute values of each parameter trend slope over a certain time window in the past, e.g. if the hematocrit level increases in the past day weight1 has a positive value equal to the slope of the trend. If the hematocrit level decreases, weight1 has a negative value corresponding to a negative / decreasing trend. The coefficients could take the value +1 or -1 respectively, when the trend is conducive to infection decline or contribute to infection worsening.

The system 100 may further comprise a calculation module 112 for calculation of a wound healing score based on the parameters detected by the one or more sensing means (104, 106) and the image acquired by the image sensing module 110. The wound healing score indicates a status of the wound healing. the wound healing score is determined based on at least one of following factors: the Pulse Oximeter Oxygen Saturation (SpO2) level of the wound 108; the hydration level of the wound 108; the wound infection risk; the size of the wound 108; the maximum width of the wound 108; the maximum height of the wound 108; the coloration of the wound 108.

In some embodiments, the wound healing score (referred to as "HealingScore") is calculated as a combination of the SpO2 level of the wound (referred to as "Sp02Level"), the hydration level of the wound (referred to as "Tissue Hydration Level"), and the infection risk score (referred to as "InfectionRiskScore"), the maximum width of wound area (referred to as "SizeX'), the maximum hight of the wound area (referred to as "SizeY"), the area of the wound (referred to as "WoundArea"), the amount of red detected in the wound bed after calibration based on the corner markers (referred to as "WoundRed") as below:

$$HealingScore = SPO2Level \otimes TissueHydrationLevel \otimes InfectionRiskScore \otimes SizeX \otimes$$
$$SizeY \otimes Wound\ Area \otimes WoundRed \qquad [3]$$

In an embodiment, the formula could be implemented as below:

$$HealingScore = SPO2Level*coeff1*weight1 + TissueHydrationLevel* coeff2*weight2 +$$
$$InfectionRiskScore\ coeff3*weight3 + SizeX *coeff4*weight4 + SizeY* coeff5*weight5 +$$
$$Wound\ Area * coeff6*weight6 + WoundRed*coeff7*weight7. \qquad [4]$$

where the weights could indicate the absolute value of each corresponding trend slope, and the coefficients could take the value +1 or -1 respectively, when the trend is conducive to healing or contribute to its deterioration.

By using data relating to multiple contributory factors, particularly from all seven factors discussed above, a better understanding of the relevant factors that are contributing to the wound healing of the subject can be achieved.

The system 100 may further comprises a processor 122 configured to internally communicate with the wound infection determination module 120, the calculation module 112 and the image processing module 114. In some embodiments, the wound infection determination module 120, the calculation module 112 and the image processing module 114 may be formed as part of the processor 122, for instance, as a sub-module of the processor 122. The processor 122 is configured for receiving the image data from the image sensing module 110 via an image data interface. The processor 122 may be configured for receiving sensing data from the sensing means (104, 106) via sensing data communication interface. The processor 122 may be further configured for communicating with a memory 130 via data communication interface. The memory 130 may comprise instruction data representing a set of instructions which configures the processor 160 to, during operation of the system 100, perform a computer-implemented method 200 as mentioned below. The processor 122 may comprise one or more processors, processing units, multi-core processors or modules that are configured or programmed to control the system 100 in the manner described herein. In particular implementations, the processor 122 may comprise a plurality of software and/or hardware modules that are each configured to perform, or are for performing, individual or multiple steps of the method described herein.

The system 100 may further comprise an output 118 for outputting the calculated wound healing score. The output 118 may be any types of output device, such as a display, for enabling outputting the calculated healing score.

In some embodiments, the system 100 may be embodied as, or in, a device or apparatus, such as a server, workstation, imaging apparatus or mobile device. The modules and/or the components of the system 100, such as the image sensing module 110, the calculation module 112, may be either located within said device or apparatus as an internal part of the device or apparatus, or located outside the device or apparatus as an external part of the device of apparatus. The device or apparatus may comprise one or more microprocessors or computer processors which execute appropriate software. The processor of the system may be embodied by one or more of these processors. The software may have been downloaded and/or stored in a corresponding memory, e.g., a volatile memory such as RAM or a non-volatile memory such as Flash. The software may comprise instructions configuring the one or more processors to perform the functions described with reference to the processor of the system. Alternatively, the functional units of the system, e.g., the image data interface, the user input interface and the processor, may be implemented in the device or apparatus in the form of programmable logic, e.g., as a Field-Programmable Gate Array (FPGA). The image data interface and the optional user input interface may be implemented by respective interfaces of the device or apparatus. In general, each functional unit of the system may be implemented in the form of a circuit. It is noted that the system 100 may also be implemented in a distributed manner, e.g., involving different devices or apparatuses. For example, the distribution may be in accordance with a client-server model, e.g., using a server and a thin-client PACS workstation.

In some embodiment, the system 100 further comprises communication module configured for transmitting the information, such as the wound healing score, to another system or device. For instance, the system 100 may transmit the wound healing score to a mobile device of the caregiver medical staff involved in the supervision of the patient regarding the patient current healing status, e.g. current Healing Score and details on all the contributing aspects above, such as wound area and coloration, tissue oxygenation, hydration, chance for infection determined based on haematocrit, temperature and PH levels and trends, patient healing status over a period of time specified by the users.

Furthermore, depending on the calculated values and trends of the wound healing score, the system 100 may provide suggestions or alerts regarding patient care. Examples of such suggestions or alert can be:

A. Bandage needs to be changed
B. Infection onset detected
C. Infection developing
D. Significant inflammation since yesterday
E. Wound size significantly increased since day x

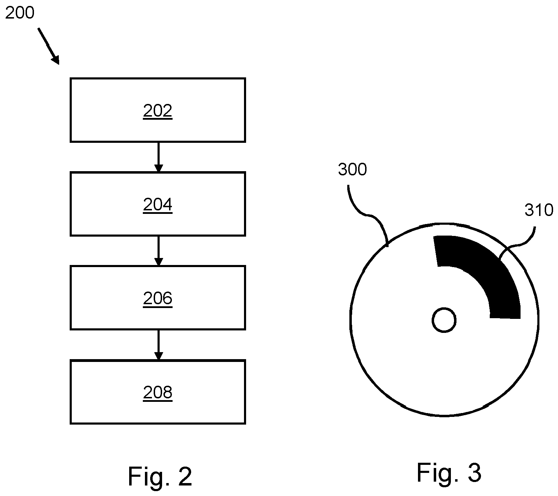

Figure 2 is a flow chart of an example of a computer-implemented method 200 for for monitoring wound healing of a subject. The method 200 comprises, at step 202, detecting one or more parameters of a wound 108 within a region of interest of the subject by one or more sensing means (104, 106) being in contact with the region of interest. The method further comprises, at step 204, capturing an image of the region of interest by an image sensing module 110. The method further comprises, at step 206, calculating a wound healing score based on the parameters detected by the one or more sensing means (104, 106) and the image acquired by the image sensing module 110, wherein the wound healing score indicating a status of the wound healing. Finally, the method 200 comprises, at step 208, outputting the calculated wound healing score.

The method 200 may be implemented on a computer as a computer implemented method, as dedicated hardware, or as a combination of both. As also illustrated in Fig. 3, instructions for the computer, e.g., executable code, may be stored on a computer readable medium 300, e.g., in the form of a series 310 of machine readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 3 shows an optical disc 300.

The term "module", as used herein is intended to include a hardware component, such as a processor or a component of a processor configured to perform a particular function, or a software component, such as a set of instruction data that has a particular function when executed by a processor.

Examples, embodiments or optional features, whether indicated as non-limiting or not, are not to be understood as limiting the invention as claimed.

It will be appreciated that the invention also applies to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

It should be noted that the above-mentioned embodiments illustrate rather than limit the invention, and that those skilled in the art will be able to design many alternative embodiments without departing from the scope of the appended claims in practicing the claimed invention, from a study of the drawings, the disclosure and the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

**Claims**

1. A system (100) for monitoring wound healing of a subject, the system comprising:

    - a strip (102) for covering a region of interest of the subject comprising one or more sensing means (104, 106) being in contact with the region of interest; wherein the region of interest comprises a wound (108), and wherein the one or more sensing means (104, 106) is configured for detecting one or more parameters of the wound (108);
    - an image sensing module (110) for capturing an image of the region of interest;
    - a calculation module (112) for calculation of a wound healing score based on the parameters detected by the one or more sensing means (104, 106) and the image acquired by the image sensing module (110), said wound healing score indicating a status of the wound healing and
    - an output module (118) for outputting the calculated wound healing score.

2. The system according to claim 1, wherein the one or more sensing means (104, 106) comprising:

   - a first sensing means (104) for detecting PH level of the wound (108);
   - a second sensing means (106) for detecting temperature level of the wound (108).

3. The system according to claim 1 or 2, wherein the colour of the first sensing means (104) is altered based on the detected PH level of the wound, and the colour of the second sensing means (106) is altered based on detected temperature level of the wound.

4. The system according to any of claims 1-3, wherein the system (100) further comprises an image processing module (114) for processing the image acquired by the image sensing module (110) to derive a plurality of factors contributing to the determination of the wound healing score.

5. The system according to claim 4, wherein the plurality of factors comprises:

   - a Pulse Oximeter Oxygen Saturation (SpO2) level of the wound (108);
   - a hydration level of the wound (108);
   - a haematocrit level of the wound (108);
   - the colour of the first and the second sensing means (104, 106);
   - the location of the wound (108) within the region of interest of the subject;
   - the size of the wound (108);
   - the maximum width of the wound (108);
   - the maximum height of the wound (108);
   - the coloration of the wound (108).

6. The system according to any of preceding claims 1-5, wherein the system (100) further comprises a wound infection determination module (120) configure for calculating a wound infection risk score indicating the wound infection risk.

7. The system according to claim 6, wherein the wound infection risk score is calculated by the wound infection determination module (120) based on at least one of the following factors:

   - a haematocrit level of the wound (108) determined by the image processing module (114);
   - a PH level of the wound (108) detected by the first sensing means (104);
   - a temperature level of the wound (108) detected by the second sensing means (106).

8. The system according to claim 7, wherein a weight is assigned to the at least one of the haematocrit level of the wound, the PH level of the wound, and the temperature level of the wound,
   wherein the weight of a given parameter is determined based on the contribution of the given parameter to the cause of the wound infection.

9. The system according to claim 6, wherein the wound healing score is determined based on at least one of following factors:

   - the Pulse Oximeter Oxygen Saturation (SpO2) level of the wound (108);
   - the hydration level of the wound (108);
   - the wound infection risk;
   - the size of the wound (108);
   - the maximum width of the wound (108);
   - the maximum height of the wound (108);
   - the coloration of the wound (108).

10. The system according to claim 9, wherein a weight is assigned to the at least one of the following parameters: the Pulse Oximeter Oxygen Saturation (SpO2) level of the wound (108); the hydration level of the wound (108); the wound infection risk; the size of the wound (108); the maximum width of the wound (108); the maximum height of the wound (108); and the coloration of the wound (108),
   wherein the weight of a given parameter is determined based on the contribution of the given parameter to the impact of the wound healing.

11. The system according to claim 1, wherein the strip (102) is a transparent strip or semi-transparent strip configured for allowing the wound (108) being visible and detectable by the image sensing module (110).

12. The system according to claim 1, wherein the image sensing module (110) comprises:

- a light source configured for emitting a light signal with various wavelengths to the region of interest;
- an infrared camera configured for detecting one or more parameters of the wound (108).

13. The system according to claim 1, wherein the system (100) further comprises a communication module (130) configured for transmitting information to a second system (400) including at least one of:

- the wound healing score of the subject;
- the wound healing score of the subject over a period of time;
- suggestion or alert message corresponding to the wound healing score of the subject.

14. A computer-implemented method (200) for monitoring wound healing of a subject, the method comprising:

- detecting (202) one or more parameters of a wound (108) within a region of interest of the subject by one or more sensing means (104, 106) being in contact with the region of interest;
- capturing (204) an image of the region of interest by an image sensing module (110); and
- calculating (206) a wound healing score based on the parameters detected by the one or more sensing means (104, 106) and the image acquired by the image sensing module (110), wherein the wound healing score indicating a status of the wound healing; and
- outputting (208) the calculated wound healing score.

15. A computer program product (300) comprising instructions for causing a processor system to perform the method according to claim 14.

Fig. 1

200

202

204

206

208

Fig. 2

300

310

Fig. 3

Europäisches
Patentamt

European
Patent Office

Office européen
des brevets

# EUROPEAN SEARCH REPORT

Application Number

EP 17 20 7468

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/000407 A1 (SAXBY CARL [GB] ET AL) 5 January 2017 (2017-01-05) * abstract * * paragraphs [0003] - [0005], [0008], [0012], [0016], [0017], [0030], [0061] - [0066], [0086] - [0090], [0110] - [0117] * | 1-15 | INV. G16H40/63 A61B5/00 G16H50/30 |
| Y | WO 2014/161036 A1 (UNIV SOUTH AUSTRALIA [AU]) 9 October 2014 (2014-10-09) * abstract * * paragraphs [0009], [0010], [0015] - [0018], [0037], [0039], [0062], [0076], [0077], [0080], [0128], [0129], [0137] - [0139] * * paragraphs [0199] - [0203], [0307], [0308]; examples 10, 13,15 * | 1-15 | |
| Y | US 2015/119721 A1 (PEDERSEN PEDER C [US] ET AL) 30 April 2015 (2015-04-30) * abstract * * paragraphs [0005] - [0007], [0032] - [0037], [0044], [0111] - [0117] * | 1-15 | TECHNICAL FIELDS SEARCHED (IPC) G16H A61B |
| Y | ROBERT J. MEIER ET AL: "Simultaneous Photographing of Oxygen and pH In Vivo Using Sensor Films", ANGEWANDTE CHEMIE INTERNATIONAL EDITION, vol. 50, no. 46, 11 November 2011 (2011-11-11), pages 10893-10896, XP55268776, ISSN: 1433-7851, DOI: 10.1002/anie.201104530 * page 10893, column 1, paragraph 2 - column 2, paragraph 1 * * page 10894, column 2, paragraph 1 - page 10895, column 1, paragraph 4 * | 1-15 | |

-/--

The present search report has been drawn up for all claims

| Place of search | Date of completion of the search | Examiner |
|---|---|---|
| Munich | 30 May 2018 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 17 20 7468

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | XISHENG CHEN ET AL: "Drug-Porous Silicon Dual Luminescent System for Monitoring and Inhibition of Wound Infection", ACS NANO, vol. 11, no. 8, 17 July 2017 (2017-07-17), pages 7938-7949, XP055479642, US ISSN: 1936-0851, DOI: 10.1021/acsnano.7b02471 * abstract * * page 7938, columns 1,2 * * page 7942, column 2, paragraph 2 - page 7944, column 1, paragraph 2; figure 4 * * page 7944, column 2, paragraph 3 - page 7945, column 1, paragraph 1 * ----- | 1-15 | |
| Y | EP 3 054 389 A2 (NXP BV [NL]) 10 August 2016 (2016-08-10) * abstract * * paragraphs [0005], [0015], [0017], [0019], [0021], [0022], [0028] - [0031], [0033], [0040] * ----- | 1-15 | |

TECHNICAL FIELDS
SEARCHED (IPC)

The present search report has been drawn up for all claims

| Place of search | Date of completion of the search | Examiner |
|---|---|---|
| Munich | 30 May 2018 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 20 7468

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2018

| Patent document cited in search report | Publication date | Patent family member(s) | | Publication date |
|---|---|---|---|---|
| US 2017000407 A1 | 05-01-2017 | AU | 2015208300 A1 | 04-08-2016 |
| | | CA | 2937579 A1 | 30-07-2015 |
| | | CN | 106455984 A | 22-02-2017 |
| | | EP | 3096679 A1 | 30-11-2016 |
| | | JP | 2017510319 A | 13-04-2017 |
| | | RU | 2016133454 A | 01-03-2018 |
| | | US | 2017000407 A1 | 05-01-2017 |
| | | WO | 2015110411 A1 | 30-07-2015 |
| WO 2014161036 A1 | 09-10-2014 | AU | 2014246657 A1 | 19-11-2015 |
| | | AU | 2018202280 A1 | 26-04-2018 |
| | | WO | 2014161036 A1 | 09-10-2014 |
| US 2015119721 A1 | 30-04-2015 | US | 2015119721 A1 | 30-04-2015 |
| | | WO | 2015066297 A1 | 07-05-2015 |
| EP 3054389 A2 | 10-08-2016 | CN | 105852802 A | 17-08-2016 |
| | | EP | 3054389 A2 | 10-08-2016 |
| | | US | 2016228049 A1 | 11-08-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5277181 A

**Non-patent literature cited in the description**

- **J. M. GOLDMAN.** Noninvasive measurement of hematocrit and hemoglobin content by differential optical analysis'' and ''Masimo signal extraction pulse oximetry. *Journal of Clinical Monitoring and Computing,* 2000, vol. 16 (7